# EUROPEAN PATENT SPECIFICATION

(11) **EP 3 383 335 B1**
(45) Date of publication and mention of the grant of the patent: **24.02.2021**
(21) Application number: 16816009.1
(22) Date of filing: 10.11.2016
(51) Int. Cl.: A61F 13/511, A61F 13/62

(54) **NONWOVEN CONNECTION PART ON DISPOSABLE DIAPERS AND THE PRODUCTION METHOD OF SAID CONNECTION PART**
VERBINDUNGSTEIL AUS VLIESSTOFF AUF WEGWERFWINDELN UND VERFAHREN ZUR HERSTELLUNG DIESES VERBINDUNGSTEILS
PARTIE DE LIAISON DE NON-TISSÉ SUR COUCHES JETABLES ET PROCÉDÉ DE PRODUCTION DE LADITE PARTIE DE LIAISON

(30) Priority: 04.12.2015 TR 201515483
(43) Date of publication of application: 10.10.2018
(73) Proprietor: Sareks Ambalaj Sanayi Ve Ticaret A.S., Tekirdag (TR)
(72) Inventor: IPEKLER, Serhat, Istanbul (TR)
(74) Representative: Kaya, Erdem
(86) International application number: PCT/TR2016/050430
(87) International publication number: WO 2017/095348

(56) References cited:
- EP-A1- 1 181 873
- EP-A2- 1 216 679
- WO-A1-99/14045
- US-A- 5 615 460

## Description

### TECHNICAL FIELD

The present invention relates to disposable diaper embodiments like baby diaper and patient diaper.

### PRIOR ART

Disposable diapers like baby diapers, sanitary diapers and patient diapers are among the needs of our daily lives since they are frequently needed and since they are easy to use. Particularly in baby diapers, it is important that said diapers are not accidentally opened due to the motions of babies or small children, body temperature, etc., and moreover, the diaper shall have a high absorbance.

In use, the resilient male connection part is fixed to the front section of the sanitary diaper, and the sanitary diaper is bonded to the user. The male and female connection parts are used in open/close structure with each other. The protrusions like hook provided on the male connection part engage onto the female connection elements in nonwoven form provided at the front section. The front section which is in nonwoven form is embodied in bonded structure, and during the opening closing process, the front section shall not be delaminated. At the same time, in order to provide opening/closing easiness, the front section shall be at least partially resilient. Thus, the connection proportion and manner of the layers forming the bonded front section gain importance.

In the invention with publication number WO2010135508A1; a bonded region embodiment in waved form provided at the front section with nonwoven structure is disclosed. There are pluralities of bonded regions embodied essentially parallel to each other.

In the patent with publication number US7806818B2; a loop member for a mechanical fastener comprises a nonwoven web, the nonwoven web having a pattern of intersecting bond lines. The pattern is characterized in that at least a portion comprises a first plurality of non-intersecting continuous bond lines and a second plurality of non-intersecting continuous bond lines, each non-intersecting continuous bond line of the first plurality intersecting each non-intersecting continuous bond line of the second plurality. The intersecting bond lines define non-bonded pattern elements, each of the pattern elements being at least partially bonded by non-linear segments of the bond lines.

In the invention with publication number US5615460; a female component is disclosed. A female component for a refastenable fastening device having an elastomeric adhesive backing and a multiplicity of fibrous elements extending from the backing. The female fastening component is formed by a method comprising the steps of: providing a first lamina comprising an elastomeric, pressure-sensitive adhesive film having a first adhesive surface and a second adhesive surface opposed to said first adhesive surface, a relaxed orientation, an elongated orientation and preferably at least two regions of differential elongation; stretching said first lamina from said relaxed orientation to said elongated orientation; contacting a second lamina comprising a nonwoven web with said first surface of said first lamina in said elongated orientation, thereby directly joining said second lamina and said first lamina to form a laminate; and relaxing said first lamina such that said second lamina is shirred to form catching regions capable of entangling the hooks of a complementary male fastening component.

In the invention with publication number WO9914045; a composite material adapted for mechanical fastener is disclosed. A composite material adapted for mechanical fastener use as the loop component complementary to a hook component. The composite material is made by laminating a film with an amorphous polymer layer to a prebonded nonwoven web, using a bond pattern different from the pattern on the nonwoven so as to provide loose filaments or fibers between the laminate bond points. Desirably, the composite also has a MVTR of at least about 100 g/m<2>/24 hours and a hydrohead of at least about 50 mbar. In use as a component of a disposable personal care product such as a disposable diaper, the loop fastener component may be substantially the entire backing to provide comfort, protection and highly variable fit.

### BRIEF DESCRIPTION OF THE INVENTION

The present invention relates to the front section embodiment in disposable diapers, for eliminating the above mentioned disadvantages and for bringing new advantages to the related technical field.

The main object of the present invention is to provide a front section embodiment where cost is reduced and where functionality is not compromised.

Another object of the present invention is to provide a front section embodiment having a higher resistance with smaller binding area.

Another object of the present invention is to provide a front section embodiment where the delamination between the layers is prevented.

Another object of the present invention is to provide a front section embodiment which will allow a comfortable / reliable opening closing system by means of the male connection element.

In order to realize all of the abovementioned objects and the objects which are to be deducted from the detailed description below, the present invention is a front section provided on a main body on a sanitary diaper and which is in layered form and where a male connection part is connected in an openable and closeable manner. Accordingly, said front section is characterized in that said front section comprises:
- said front section is formed by aligning a first nonwoven layer, a thermoplastic film layer and a second nonwoven layer respectively and
- said front section comprises pluralities of bonded regions which thermally weld at least said second nonwoven layer and said thermoplastic film layer and which form a laminated structure and embodied in a repetitive pattern in at least one of star-like, short line, curved line, waved line and elliptical forms in an independent manner from each other and the thickness of the bonded region is 1,5 mm at most,
- length of a bonded region is between 2 and 10 mm,
- the bonded regions occupy 13-20 % of the area of the front section,
- distance between the bonded region and the other adjacent bonded region is at least 1 mm and at most 10 mm.
- weight of the second nonwoven layer is between 20 and 50 g/m²; and weight of the thermoplastic film layer is between 6 and 45 g/m²,

In a preferred embodiment of the invention, a first nonwoven layer is positioned in the vicinity of the second nonwoven layer.

In a preferred embodiment of the invention, the first nonwoven layer is positioned in the vicinity of the thermoplastic film layer.

In a preferred embodiment of the invention, said front section is bonded region which ultrasonically welds at least said second nonwoven layer and said thermoplastic film layer.

In a preferred embodiment of the invention, said front section is bonded region which thermally welds at least said second nonwoven layer and said thermoplastic film layer.

In a preferred embodiment of the invention, said front section is bonded region which binds at least said second nonwoven layer and said thermoplastic film layer by means of glue.

In a preferred embodiment of the invention, the bonded regions are positioned in a manner extending outwardly from a center.

In a preferred embodiment of the invention, the bonded regions are positioned in a manner forming telescopic snow particles when brought together.

In another preferred embodiment of the invention, the bonded regions are positioned in a manner forming a diagonal-like pattern when brought together.

In another preferred embodiment of the invention, the bonded regions are positioned in a manner forming a brick-like pattern when brought together.

In another preferred embodiment of the invention, the bonded regions are positioned in a manner forming a polygonal pattern when brought together.

In another preferred embodiment of the invention, the bonded regions are each embodied in a short independent straight line form.

In another preferred embodiment of the invention, the bonded regions are each embodied in a short independent semi "S" form.

In another preferred embodiment of the invention, the bonded regions occupy 16.4 % of the area of the front section.

In another preferred embodiment of the invention, the length of the bonded region is between 2 and 10 mm.

In another preferred embodiment of the invention, the length of the bonded region is between 3 and 4 mm.

In another preferred embodiment of the invention, the thickness of the bonded region is 1.5 mm at most.

In another preferred embodiment of the invention, the distance between the bonded region and the other adjacent bonded region is at least 1 mm and at most 10 mm.

In another preferred embodiment of the invention, the weight of the thermoplastic film layer is between 6 and 20 g/m².

In another preferred embodiment of the invention, the weight of the thermoplastic film layer is between 10 and 45 g/m².

In another preferred embodiment of the invention, the weight of the thermoplastic film layer is 10 g/m².

In another preferred embodiment of the invention, a non-bonded continuous region is defined at the section remaining between at least two bonded regions.

In another preferred embodiment of the invention, the layered structure is in the form of thermoplastic film layer and the second nonwoven layer respectively.

In another preferred embodiment of the invention, the layered structure is formed by aligning the thermoplastic film layer, the first nonwoven layer and the second nonwoven layer respectively.

In another preferred embodiment of the invention, the layered structure is formed by aligning the first nonwoven layer, the thermoplastic film layer and the second nonwoven layer respectively.

In another preferred embodiment of the invention, the second nonwoven layer is in circular form and pre-bonded in a punctual manner.

### BRIEF DESCRIPTION OF THE FIGURES

In Figure 1, a general representative view of the baby diaper is given.
In Figure 2, a representative view of the side band is given.
In Figure 3, a representative view of the front section is given.

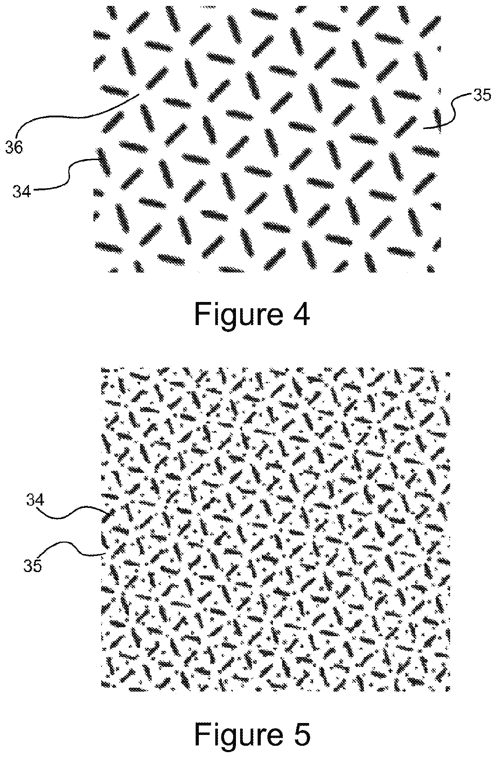

In Figure 4, a detailed representative view of the front section is given.
In Figure 5, a representative view of the alternative application of the front section is given.

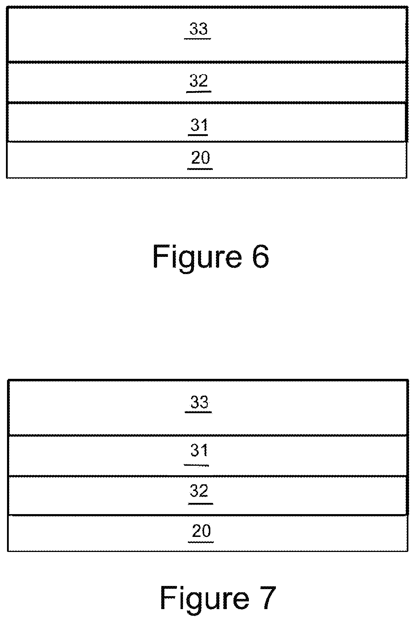

In Figure 6, a representative view of the layer embodiment of the front section is given.
In Figure 7, a representative view of the alternative layer embodiment of the front section is given.

### REFERENCE NUMBERS

- 10: Sanitary diaper
- 20: Main body
- 30: Front section
31 Thermoplastic film layer
32 First nonwoven layer
33 Second nonwoven layer
34 Bonded region
35 Non-bonded region
36 Center
- 40: Side band
41 Main carrier
42 Male connection part
- CD: direction: →
- MD: direction: ↑

### DETAILED DESCRIPTION OF THE INVENTION

In this detailed description, the subject matter disposable diaper (10) front section (30) is explained with references to examples without forming any restrictive effect only in order to make the subject more understandable.

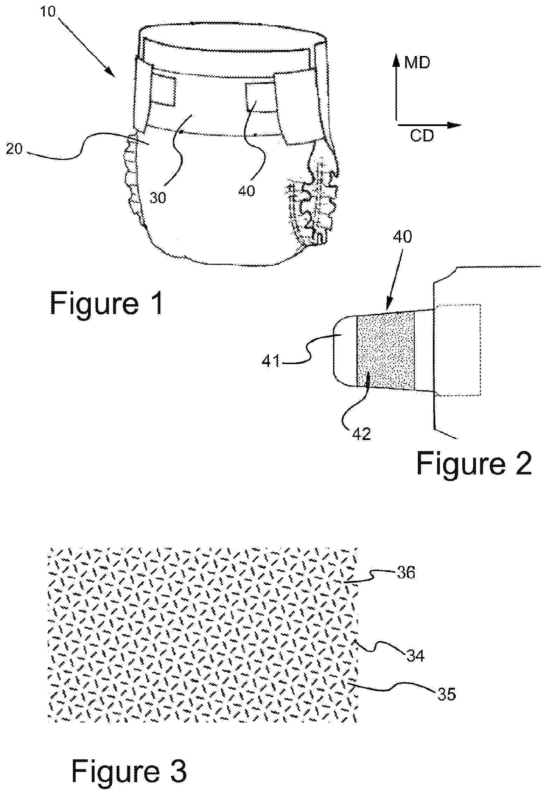

With reference to Figure 1, in order to form a diaper (10), there is a main body (20) and there is a side band (40) connected to said main body (20). When the sanitary diaper (10) is in open position, the direction extending from the side band (40) towards the main body (20) is named as the CD direction (→), and the direction from the side band (40) towards the sanitary diaper (10) is named as the MD direction (↑). Said main body (20) comprises a front section (30). Said front section (30) essentially has textile surface and/or it is in loop structure formed by textile (it is named nonwoven in the related art, and it will be named as nonwoven in the present invention).

Said front section (30) has a layered structure, and a thermoplastic film layer (31) comprises a second nonwoven layer (33) extending on said thermoplastic film layer (31). In the preferred application, a first nonwoven layer (32) is positioned between the thermoplastic film layer (31) and the second nonwoven layer (33). The thermoplastic film layer (31) comprises at least one bonded region (34) which defines the regions where at least two of the first nonwoven layer (32) and the second nonwoven layer (33) are connected to each other, and at least one non-bonded region (35) which defines the regions remaining outside said bonded region (34). There is at least one center (36) defined between the ends of the bonded regions (34) which are close to each other. The front section (30) may comprise the second nonwoven layer (33) and the thermoplastic film layer (31); the second nonwoven layer (33) may also comprise thermoplastic film layer (31) and the first nonwoven layer (32).

Said side band (40), made of at least one thermoplastic based material, generally comprises a main carrier (41) and a male connection part (42) connected onto said main carrier (41).

The thermoplastic film layer (31) is produced by using polyethylene (hereafter, it will be called PE), polypropylene (hereafter it will be called PP) or a mixture of them which are suitable for the casting or blowing film production method.

The first nonwoven layer (32) and the second nonwoven layer (33) are preferably made of nonwoven material. Nonwoven is essentially a thermoplastic based material. The nonwoven material used in the present invention is preferably made of thermoplastic PP material. In alternative embodiments, PP/PE, PET, viscose and derivates can be used.

The first nonwoven layer (31) preferably has PP structure, and it may be spun-bond, melt-blown, spun-melt or the mixture of them based on the nonwoven production method. However, there is no obligation to provide melt-blown or melt structure therein. The second nonwoven layer (33) whereto the male connection part (42) is to be held is preferably PP-based, and it preferably consists of carded, spun-bond, spun-lace, air through, air laid structures or the mixture of them depending on the nonwoven production method.

In the present invention, the thermoplastic film layer (31), the first nonwoven layer (32) and the second nonwoven layer (33) can be connected by means of at least one of the connection methods like ultrasonic and/or thermal and/or glue through the bonded regions (34). In the preferred application, ultrasonic method is used for connection.

The thermoplastic film layer (31) functions as the carrier layer. Another function of the thermoplastic film layer (31) is to minimize air permeability. The second nonwoven layer (33) provides the male connection part (42) to be held to the diaper (10) and provides a safe open-close system to be formed. The thermoplastic film layer (31), the first nonwoven layer (32) and the second nonwoven layer (33) are connected to each other through pluralities of regions (34).

The bonded regions (34) are positioned on the front section (30) in an independent manner from each other and essentially they have a short structure and they have a repeating form at a predetermined pattern. Each bonded region (34) comprises at least one of star-like, short line, curved line, waved line and elliptical forms. Preferably the bonded region (34) is in line form. In the alternative embodiment, the bonded regions (34) are in elliptical structure. The length of a bonded region (34) is between 2 and 10 mm. In the preferred application, the length of a bonded region (34) is between 3 and 4 mm. The thickness of a bonded region (34) is at most 1.5 mm. The thickness of a bonded region (34) is preferably at most 1 mm.

When pluralities of bonded regions (34) are brought together, they are positioned in a manner forming telescopic snow particles when viewed from the outer side. The bonded regions (34) do not intersect and contact with each other, and they have a non-continuous structure. Another characteristic of the bonded regions (34) is that they are not encircled with contour. There is a pre-calculated distance between at least two bonded regions (34). There is the non-bonded region (35) within this distance in between. The non-bonded regions (35) are defined at least at the section essentially remaining between at least two bonded regions (35). The non-bonded regions (35) have a continuous structure.

The bonded regions (34) are such that the bonded regions (34) extend outwardly from said center (36) in one of the positioning styles on the front section (30). Preferably when the bonded regions (34) extend outwardly from the center (36), a hexagonal appearance is formed when the finishing ends of the bonded regions (34) are joined. When the number of bonded regions (34) extending outwardly from the center (36) increases, a circular appearance is formed when the finishing ends of the bonded region (34) are joined.

The distance between the end section of a bonded region (34) and the end section of the other adjacent bonded region (34) is at least 1 mm and at most 10 mm. In the preferred application, the distance between the end section of the bonded region (34) and the end section of the other adjacent bonded region (34) is at least 1.5 mm and at most 8 mm. In the most preferred application, the distance between the end section of the bonded region (34) and the end section of the other adjacent bonded region (34) is at least 2 mm and at most 5 mm.

At the front section (30) of the bonded regions (34), the proportion of the occupied area in MD (↑) and CD (→) direction, in other words, the application density in total is between 13-20 %. In the preferred embodiment, the application density is 15-18 %. In the most preferred embodiment, the application density is 16-17 %. In the specific application, it is 16.4 %. The bonded region (34) allows a more resistant female - male connection thanks to its shape and its proportion in the total area.

In the present invention, thanks to a front section (30) embodiment which does not intersect with each other and which is flatter and whose bonded region (34) proportion is reduced, the front section (30) is prevented from pilling during opening of the male connection part (42) from the front section (30) and during re-connection thereof. Particularly during the following opening and closing processes, the deterioration of the functionality of the front section (30) due to this pilling effect is prevented. At the same time, thanks to its structure, a much softer and comfortable structure is obtained.

In the first application of the present invention, the thermoplastic film layer (31) is flowed and plastered onto the first nonwoven layer (32) preferably by means of plastering / covering extrusion method, and the double layered structure is obtained. The two layered structure and the second nonwoven layer (33) obtained thereafter are joined by using at least one of ultrasonic or thermal or glue lamination methods, and the front section (30) is obtained. Preferably ultrasonic method is used for joining. During lamination, the thermoplastic film layer (31) is positioned at the bottom. In the first application, the layer arrangement forming the front section (30) is as follows: thermoplastic film layer (31)-first nonwoven layer (32)-second nonwoven layer (33).

In the second application of the present invention, the thermoplastic film layer (31) is flowed and plastered onto the first nonwoven layer (32) preferably by means of plastering / covering extrusion method, and the double layered structure is obtained. The two layered structure and the second nonwoven layer (33) obtained thereafter are joined by using at least one of ultrasonic or thermal or glue lamination methods, and the front section (30) is obtained. Preferably ultrasonic method is used for joining. During lamination, the thermoplastic film layer (31) is positioned in the middle. In the second application, the layer arrangement forming the front section (30) is as follows: first nonwoven layer (32)-thermoplastic film layer (31)-second nonwoven layer (33). In other words, the polyethylene and/or polypropylene structure can be provided in the middle.

In the third application of the present invention, the thermoplastic film layer (31) is flowed and plastered onto the second nonwoven layer (33) preferably by means of extrusion method. Afterwards, an additional drive effect is applied to the holding of the double structure to each other by using at least one of ultrasonic or thermal or glue lamination methods, and the front section (30) is obtained. Preferably ultrasonic method is used for joining.

In the fourth application of the present invention, circular and/or punctual pre-bonding process is applied to the second nonwoven layer (33). Afterwards, the second nonwoven layer (33) is joined onto the first nonwoven layer (32) by flowing the thermoplastic film layer (31) by using at least one of the ultrasonic or thermal or glue lamination methods by means of the layered structure prepared beforehand. Preferably ultrasonic method is used for joining.

In the third application of the present invention, the thermoplastic film layer (31) used can be PP or PP/PE mixture. In case it is PP/PE mixture, it is a PP dominant PP/PE mixture. The thermoplastic film layer (31) used in the first and second application of the present invention can be in PE or PP/PE mixture form. In case it is PP/PE mixture, it is a PE dominant PP/PE mixture. As the PE material derivative, at least one of low density polyethylene (LDPE), intermediate density polyethylene (MDPE), high density polyethylene (HDPE), linear low density polyethylene (LLDPE) and/or the mixture of them can be used.

In the present invention, the weight of the first nonwoven layer (32) is a value between 8 and 15 g/m². In the preferred application, a first nonwoven layer (32) of 10 g/m² is used. The weight of the thermoplastic film layer (31) is a value between 6 and 45 g/m². In an application of the present invention, the weight of the thermoplastic film layer (31) is a value between 6 and 20 g/m². In an alternative application, the weight of the thermoplastic film layer (31) can be between 10 and 45 g/m². In the preferred application, it is 10 g/m². The weight of the second nonwoven layer (33) is between 20 and 50 g/m². In the preferred application, a second nonwoven layer (33) of 27 g/m² is used. In an alternative embodiment of the present invention, a second nonwoven layer (33) of 20-50 g/m² is used. In the preferred application, in the alternative embodiment, a second nonwoven layer (33) of 40 g/m² is used.

When ultrasonic method is applied, at least one of the patterns like snow particle, polygonal, brick arrangement, star-like, diagonal-like patterns can be applied by bringing together the bonded regions (34) which are in cut short line form. Each bonded region (34) can have short straight line form independent from each other, and it can have independent curved or semi "S" form. In different applications, similar forms and/or different pattern applications can also be used. The bonded regions (34) are positioned in an angled manner with respect to each other. In another embodiment, the bonded regions (34) can also be positioned in a sequenced manner with respect to each other.

In case the bonded region proportion (34) of the front section (30) is between 13-20 % and in case the bonded regions (34) are in independent and iterative form and in case the bonded regions (34) are applied within mentioned dimensions, a higher resistance value is obtained since the bonding proportion is reduced. Thanks to this structure, both functional and comfortable and safe opening-closing process can be provided by means of the male connection part (42), and thus, front section (30) usage is provided comprising less dense fiber structure and low cost nonwoven form. At the same time, a problem like delamination is not faced between the joined parts.

In case a part, having width of 25 mm and which is to be cut in CD (→) direction through the front section (30), is drawn in the tension-meter device with speed of 500 mm/minute, the resistance (Fmax N/25 mm) of the front section (30) in MD direction is at least 30 N, and the resistance (Fmax N/25 mm) thereof in CD direction is at least 10 N.

At the same time, the hook peel test, where the male connection element (40) is positioned on the front section (30) and where the opening movement of the end user and the drawing movement of the baby are symbolized, is at the level of at least 3 N depending on the type of the male connection element (40) of the front section (30) in CD direction (→) and depending on the drawing speed (preferably 305 mm/minute) in the tension-meter, and in the same manner, the shear test is at the level of at least 30 N.

The first nonwoven layer (32) and the second nonwoven layer (33) are permitting air passage, and they are made of nonwoven comprising material manufactured by means of interlining, spun-bond, carded, spun-lace, spun-bond / carded mixture and the mixture of these structures and similar production method. The nonwoven materials are substantially permeable. Since thermoplastic film layer (31) is used at the front section (30) and particularly, since polyethylene is used as the thermoplastic film layer (31), the air permeability of the front section (30) is substantially reduced. Therefore, any additional or important modification like vacuuming in the machines is not needed during any process step to be applied to the front section (30). Thus, the costs are reduced. The air permeability value of the front section (30) is at least 46 mm/sec. The air permeability test is realized by applying vacuum at a pressure of 100 Pa on a front section (34) with size 20 cm².

The protection scope of the present invention is set forth in the annexed Claims and cannot be restricted to the illustrative disclosures given above, under the detailed description. It is because a person skilled in the relevant art can obviously produce similar embodiments under the light of the foregoing disclosures, without departing from the main principles of the present invention.

## Claims

1. A front section (30) provided on a main body (20) on a sanitary diaper (10) and which is in layered form and where a male connection part (42) is connected in an openable and closeable manner; **characterized in that** said front section (30) comprises:
- said front section (30) is formed by aligning a first nonwoven layer (32), a thermoplastic film layer (31) and a second nonwoven layer (33) respectively and
- said front section (30) comprises pluralities of bonded regions (34) which thermally weld at least said second nonwoven layer (33) and said thermoplastic film layer (31) and which form a laminated structure and embodied in a repetitive pattern in at least one of star-like, short line, curved line, waved line and elliptical forms in an independent manner from each other and the thickness of the bonded region (34) is 1.5 mm at most,
- length of a bonded region (34) is between 2 and 10 mm,
- the bonded regions (34) occupy 13-20 % of the area of the front section (30),
- distance between the bonded region (34) and the other adjacent bonded region (34) is at least 1 mm and at most 10 mm.
- weight of the second nonwoven layer (33) is between 20 and 50 g/m²; and weight of the thermoplastic film layer (31) is between 6 and 45 g/m².

2. A front section (30) according to claim 1, wherein the bonded regions (34) are positioned in a manner extending outwardly from a center (36) that defined between the ends of the bonded regions (34) which are close to each other in front section (30).

3. A front section (30) according to claim 1, wherein the bonded regions (34) are positioned in a manner forming telescopic snow particles when brought together.

4. A front section (30) according to claim 1, wherein the bonded regions (34) are each embodied in a short independent straight line form.

5. A front section (30) according to claim 1, wherein the bonded regions (34) occupy 16.4 % of the area of the front section (30).

6. A front section (30) according to claim 1, wherein the length of the bonded region (34) is between 2 and 10 mm.

7. Afront section (30) according to claim 1, wherein a non-bonded continuous region (35) is defined at the section remaining between at least two bonded regions (35).

8. A front section (30) according to claim 1, wherein the layered structure is formed by aligning the thermoplastic film layer (31), the first nonwoven layer (32) and the second nonwoven layer (33) respectively.

9. A front section (30) according to claim 1, wherein the second nonwoven layer (33) is in circular form and pre-bonded in a punctual manner.

## Patentansprüche

1. Mehrschichtig ausgestalteter und auf einem Grundkörper (20) einer Hygienewindel (10) vorgesehener Frontabschnitt (30), an welchem ein männliches Verbindungsstück (42) auf öffenbare und verschließbare Weise verbunden ist, **dadurch gekennzeichnet, dass** der Frontabschnitt (30):
- der Frontabschnitt (30) durch Ausrichten einer ersten Vliesschicht (32), einer thermoplastischen Filmschicht (31) bzw. einer zweiten Vliesschicht (33) gebildet ist, und
- der Frontabschnitt (30) mehrere gebundene Bereiche umfasst (34), die mindestens die zweite Vliesschicht (33) und die thermoplastische Filmschicht (31) thermisch miteinander verschweißen, eine laminierte Struktur bilden und in mindestens einem sich wiederholenden Muster ausgebildet in Sternform oder in Form von Kurzstrichen, gekrümmten Linien, gewellten Linien oder elliptischen Formen in voneinander unabhängiger Weise ausgestaltet sind, wobei die Dicke des gebundenen Bereichs (34) höchstens 1.5 mm beträgt,
- die Länge eines gebundenen Bereichs (34) zwischen 2 und 10 mm liegt,
- die gebundenen Bereiche (34) flächenmäßig 13-20% des Frontabschnitts (30) einnehmen,
- der Abstand zwischen dem gebundenen Bereich (34) und dem diesem benachbart angeordneten gebundenen Bereich (34) mindestens 1 mm und höchstens 10 mm beträgt,
- das Gewicht der zweiten Vliesschicht (33) zwischen 20 und 50 g/m² und das Gewicht der thermoplastischen Filmschicht (31) zwischen 6 und g/m² liegt.

2. Frontabschnitt (30) nach Anspruch 1, wobei die gebundenen Bereiche (34) in einer sich von einem zwischen den im Frontabschnitt (30) nahe beieinander liegenden Enden der gebundenen Bereiche (34) abgegrenzten Zentrum (36) nach außen hin erstreckenden Weise angeordnet sind.

3. Frontabschnitt (30) nach Anspruch 1, wobei die gebundenen Bereiche (34) so angeordnet sind, dass sie, wenn zusammengeführt, teleskopische Schneeflocken bilden.

4. Frontabschnitt (30) nach Anspruch 1, wobei die gebundenen Bereiche (34) jeweils in einer kurzen unabhängigen geraden Linienform ausgestaltet sind.

5. Frontabschnitt (30) nach Anspruch 1, wobei die gebundenen Bereiche (34) flächenmäßig 16.4% des Frontabschnitts (30) einnehmen.

6. Frontabschnitt (30) nach Anspruch 1, wobei die Länge des gebundenen Bereichs (34) zwischen 2 und 10 mm liegt.

7. Frontabschnitt (30) nach Anspruch 1, wobei an dem zwischen mindestens zwei gebundenen Bereichen (35) verbleibenden Abschnitt ein nicht gebundener kontinuierlicher Bereich (35) abgegrenzt ist.

8. Frontabschnitt (30) nach Anspruch 1, wobei die Schichtstruktur durch Ausrichten der thermoplastischen Filmschicht (31), der ersten Vliesschicht (32) bzw. der zweiten Vliesschicht (33) gebildet wird.

9. Frontabschnitt (30) nach Anspruch 1, wobei die zweite Vliesschicht (33) in Kreisform ausgestaltet und punktförmig vorgebunden ist.

## Revendications

1. Une section avant (30) prévue sur un corps principal (20) sur une couche hygiénique (10) et qui est sous forme de couches et où une partie de connexion mâle (42) est reliée d'une manière pouvant être ouverte et fermée; **caractérisé en ce que** ladite section avant (30) comprend:
- ladite section avant (30) est formée en alignant respectivement une première couche non tissée (32), une couche de film thermoplastique (31) et une seconde couche non tissée (33) et
- ladite section avant (30) comprend une pluralité de régions liées (34) qui soudent thermiquement au moins ladite deuxième couche non tissée (33) et ladite couche de film thermoplastique (31) et qui forment une structure stratifiée et incarnée dans un motif répétitif au moins l'une des formes en étoile, ligne courte, ligne courbe, ligne ondulée et elliptique d'une manière indépendante l'une de l'autre et l'épaisseur de la région liée (34) est au plus de 1.5 mm,
- la longueur d'une région liée (34) est comprise entre 2 et 10 mm,
- les régions liées (34) occupent 13-20% de la surface de la section avant (30),
- la distance entre la région liée (34) et l'autre région liée adjacente (34) est d'au moins 1 mm et d'au plus 10 mm.
- le poids de la deuxième couche non tissée (33) est compris entre 20 et 50 g / m2; et le poids de la couche de film thermoplastique (31) est compris entre 6 et 45 g / m2.

2. Section avant (30) selon la revendication 1, dans laquelle les régions liées (34) sont positionnées d'une manière s'étendant vers l'extérieur à partir d'un centre (36) défini entre les extrémités des régions liées (34) qui sont proches de chacune, autre dans la section avant (30).

3. Section avant (30) selon la revendication 1, dans laquelle les régions liées (34) sont positionnées de manière à former des particules de neige télescopiques lorsqu'elles sont rassemblées.

4. Section avant (30) selon la revendication 1, dans laquelle les régions liées (34) sont chacune réalisées sous une forme de ligne droite indépendante courte.

5. Section avant (30) selon la revendication 1, dans laquelle les régions liées (34) occupent 16,4% de la surface de la section avant (30).

6. Section avant (30) selon la revendication 1, dans laquelle la longueur de la région liée (34) est comprise entre 2 et 10 mm.

7. Section avant (30) selon la revendication 1, dans laquelle une région continue non liée (35) est définie au niveau de la section restant entre au moins deux régions liées (35).

8. Section avant (30) selon la revendication 1, dans laquelle la structure en couches est formée en alignant respectivement la couche de film thermoplastique (31), la première couche non tissée (32) et la deuxième couche non tissée (33).

9. Section avant (30) selon la revendication 1, dans lequel la deuxième couche non tissée (33) est de forme circulaire et pré-liée de manière ponctuelle.
